# FASCICULE DE BREVET EUROPEEN

(11) **EP 2 906 035 B1**
(45) Date de publication et mention de la délivrance du brevet: **13.09.2017**
(21) Numéro de dépôt: 13785539.1
(22) Date de dépôt: 09.10.2013
(51) Int. Cl.: A01N 1/02, A61J 1/20, A61B 5/15, A61M 1/02, A61J 1/10

(54) **PROCÉDÉ ET SYSTÈME À POCHES POUR CONSERVER LE SANG PLACENTAIRE**
VERFAHREN UND SYSTEM MIT BEUTELN ZUR KONSERVIERUNG VON PLAZENTABLUT
METHOD AND SYSTEM OF BAGS FOR PRESERVING PLACENTAL BLOOD

(30) Priorité: 10.10.2012 FR 1259684
(43) Date de publication de la demande: 19.08.2015
(73) Titulaire: Maco Pharma, 59420 Mouvaux (FR); Etablissement Français du Sang, 93210 La Plaine Saint Denis (FR)
(72) Inventeur: DELORME, Bruno, 59700 Marcq-en-Baroeul (FR); IVANOVIC, Zoran, 33700 Mérignac (FR)
(74) Mandataire: Sayettat, Julien Christian
(86) Numéro de dépôt international: PCT/FR2013/052410
(87) Numéro de publication internationale: WO 2014/057220

(56) Documents cités:
- EP-A2- 0 119 469
- EP-A2- 0 920 850
- WO-A1-2004/032750
- DE-A1- 10 151 343
- US-A1- 2012 213 754
- Pall Medical: "Cord Blood Collection, Processing and Freezing Bag Sets for the Cryopreservation of Blood and Cellular Components", , 1 janvier 2009 (2009-01-01), XP055016286, Extrait de l'Internet: URL:http://www.pall.com/pdfs/Medical/09268 0_CordBld_BagSes_SS.pdf [extrait le 2012-01-12]
- MICHAEL SOLOMON ET AL: "Factors influencing cord blood viability assessment before cryopreservation", TRANSFUSION, vol. 50, no. 4, 1 avril 2010 (2010-04-01), pages 820-830, XP055066774, ISSN: 0041-1132, DOI: 10.1111/j.1537-2995.2009.02491.x

## Description

L'invention concerne un procédé pour conserver le sang placentaire entier ainsi qu'un système à poches pour mettre en oeuvre un tel procédé.

Elle s'applique à la conservation à court terme du sang placentaire entier, c'est-à-dire issu du cordon ombilical et/ou placenta et avant tout traitement du sang placentaire de type réduction de volume ou isolement des cellules. Elle s'applique notamment au sang placentaire entier juste après le prélèvement.

Le sang placentaire représente une source intéressante pour la greffe de cellules souches hématopoïétiques à des patients atteints de maladies hématologiques congénitales ou acquises comme le cancer ou les leucémies. En effet, en l'absence d'un donneur de moelle osseuse adulte et compatible, la greffe de cellules issues du sang placentaire devient la seule solution. Ensuite, une compatibilité moins rigoureuse que celle des cellules adultes permet d'obtenir de bons résultats de greffe. Enfin, le prélèvement d'un sang placentaire n'est traumatisant ni pour la mère ni pour l'enfant.

Un sang placentaire contient plusieurs types de cellules parmi lesquelles on trouve (i) des cellules qui sont sans intérêt pour la greffe (les globules rouges, les plaquettes et les granulocytes) (ii) d'autres qui peuvent moduler l'effet du greffon par voie d'immuno-modulation (les lymphocytes) et finalement, (iii) des cellules impliquées stricto sensu dans la reconstitution hématopoïétique après la greffe.

Ces dernières sont représentées par une population hétérogène, majoritairement positive pour l'antigène CD34 dont on peut schématiquement distinguer deux sous-populations cellulaires : les cellules souches et les progéniteurs engagés (CFC). Les cellules souches sont très rares dans le contenu cellulaire mais leur présence est indispensable pour la longévité du greffon et le maintien de l'hématopoïèse à long terme. Ces cellules sont détectées selon leur capacité à induire une hématopoïèse humaine dans la moelle osseuse des souris immuno-déficientes (SCID Repopulating Cells, SRC). D'autre part, les progéniteurs engagés, beaucoup plus fréquents (jusqu'à 50% de la population CD34+ de sang placentaire), sont nécessaires pour la phase précoce de la reconstitution hématopoïétique après la greffe.

Dans le contexte de mise en banque de sang placentaire, il est essentiel de préserver au mieux ces deux sous-populations de cellules hématopoïétiques afin d'optimiser les effets de la greffe de sang placentaire.

En effet, pour répondre au besoin croissant en sang placentaire, des banques de sang placentaire destinées à conserver ce sang ont été créées. Après le prélèvement d'une unité de sang placentaire (USP) à l'aide d'un système à poches tel que décrit dans le document EP-1 262 202, et après une étape dite de "réduction de volume", l'unité de sang placentaire réduite est transférée dans une poche de congélation. La poche de congélation est enfin placée dans l'azote liquide pour conservation à long terme. Cette poche de congélation est généralement réalisée en éthylène-acétate de vinyle (EVA), matériau stérilisable qui résiste aux températures cryogéniques.

Les films en EVA sont perméables aux gaz, c'est pourquoi on les utilise également communément pour la fabrication des poches destinées à la conservation des plaquettes sanguines et à la culture cellulaire, comme illustré dans les documents EP-A1-0 542 221 et US 2005/0032205, respectivement.

Il a été montré que si le sang placentaire n'est pas rapidement traité après le prélèvement en vue de sa congélation, on constate une perte des progéniteurs engagés fonctionnels (Ivanovic et al. Transfusion, 2011).

Or, dans certains cas, comme notamment lorsque le lieu de prélèvement du sang placentaire, en général l'hôpital ou la maternité, et le lieu de traitement du sang placentaire sont éloignés, il est difficile de réaliser le traitement dans les 24 heures.

Pour prolonger la période de temps entre le prélèvement et le traitement du sang placentaire, Hubel et al. ont proposé d'ajouter au sang placentaire recueilli dans une poche perméable aux gaz, une solution de stockage telle que notamment la solution Normosol, Plasmalyte A, ou STM-Sav (Hubel et al. Transfusion, 2003, 43, 626-632).

De même, dans les documents WO 2009/121002 et WO 2009/120996, le sang placentaire est prélevé dans une poche en polychlorure de vinyle (PVC) perméable à l'air et additionné de la solution Hypothermosol® de Biolife Inc. Le sang est ensuite conservé à 4°C jusqu'à 72 heures.

DE 101 51 343 A1 divulgue un procédé pour la conservation du sang placentaire entier et un système à poches comprenant une poche de stockage réalisée en EVA.

Il est également connu du document WO 2004/032750 une méthode pour stabiliser à température ambiante un échantillon de sang en utilisant un récipient contenant un ou plusieurs inhibiteurs de caspase. Le récipient sous forme de tube peut être perméable, semi-perméable ou imperméable à l'oxygène. L'invention est définie dans les revendications.

L'invention propose de conserver le sang placentaire à court terme, avant son traitement, en maintenant la viabilité et la fonctionnalité d'au moins les cellules souches et progéniteurs hématopoïétiques d'une unité de sang placentaire. De plus, l'invention propose un procédé dans lequel la viabilité et la fonctionnalité des cellules souches et progéniteurs hématopoïétiques sont maintenues tout au cours du traitement de l'unité de sang placentaire, de la collecte jusqu'à la greffe.

A cet effet, selon un premier aspect, l'invention concerne un procédé pour la conservation du sang placentaire entier comprenant les étapes de :
- introduire du sang placentaire entier dans une poche de stockage barrière à l'air, ladite poche de stockage étant réalisée avec un film dont la perméabilité à l'oxygène est inférieure à 10 cm3/m2.jour.atm,
- stocker ladite poche contenant le sang placentaire entier à une température supérieure à 0°C et inférieure à 40°C, de sorte à conserver le sang placentaire entier.

Selon un deuxième aspect, l'invention concerne un système à poches pour la mise en oeuvre d'un procédé selon le premier aspect, ledit système comprenant :
- au moins une aiguille de prélèvement,
- une poche de recueil du sang placentaire en communication fluidique, par l'intermédiaire d'une première tubulure associée à un orifice d'entrée de la poche de recueil, avec ladite au moins une aiguille de prélèvement,
- une poche de stockage barrière à l'air, ladite poche de stockage étant réalisée avec un film dont la perméabilité à l'oxygène est inférieure à 10 cm³ / m².jour.atm, ladite poche étant en communication fluidique ou destinée à être mise en communication fluidique avec la poche de recueil pour pouvoir stocker le sang placentaire.

D'autres objets et avantages apparaîtront au cours de la description qui suit.
La figure 1 représente une poche de recueil d'un système à poches pour la mise en oeuvre d'un procédé selon une réalisation particulière de l'invention.
La figure 2 représente une poche de stockage d'un système à poches, destinée à être mise en communication fluidique avec la poche de recueil de la figure 1.
La figure 3 représente un système à poches obtenu après connexion de la poche de recueil de la figure 1 et de la poche de stockage de la figure 2.
La figure 4 représente un graphe montrant le maintien des progéniteurs hématopoïétiques (CFC) stockées à 4°C pendant 3 jours dans différentes conditions de stockage.
La figure 5 représente un graphe montrant le maintien des cellules positives au marqueur CD34 (CD34+) stockées à 4°C pendant 3 jours dans différentes conditions de stockage.
La figure 6 représente un graphe montrant la viabilité des CFC stockées à 4°C, entre 0 et 14 jours, en présence ou non de la solution de conservation MC01,
La figure 7 représente un graphe montrant la capacité d'expansion des cellules CD34+ et CFC, conservées à 4°C, entre 0 et 48 heures, en présence ou non de la solution de conservation MC01.
La figure 8 représente un graphe montrant le maintien des cellules nucléées totales (CNT), cellules mononuclées (CMN), CD34+ (viables) et CFC stockées à 4°C pendant 3 jours dans des poches perméables à l'air (PVC) ou non (TriC).
La figure 9 représente un graphe montrant le maintien des cellules CNT, CMN, CD34+ et CFC stockées dans des poches barrières à l'air (TriC) à 4°C pendant 3 jours en présence ou non de la solution MC01.
La figure 10 représente un graphe montrant le maintien des cellules CNT, CMN, CD34+ et CFC stockées à 4°C pendant 3 jours dans des poches en PVC sans solution de conservation (condition actuelle pour la banque d'USP) ou dans des poches barrières à l'air (TriC) avec une solution de conservation (MC01).
La figure 11 représente un graphe montrant le maintien des cellules CD34+ viables et CFC au cours du traitement des unités de sang placentaire stockées dans des poches barrières à l'air (TriC), avec ou sans solution de conservation (MC01) : à J0, à J3 après stockage à 4°C pendant 3 jours, après réduction de volume par la méthode "Sepax" (AS), après décongélation (AD), et à 6 heures après décongélation (AD+6h).
La figure 12 représente un graphe montrant le nombre de CFC / fémur (log) après greffe d'une unité de sang placentaire stockées dans les conditions de routine ou selon le procédé de l'invention.

Actuellement, le sang de cordon ou sang placentaire doit être traité dans les 24 heures suivant sa collecte. Pouvoir retarder le traitement du sang placentaire jusqu'à 72 heures après sa collecte permettrait de pouvoir collecter le sang placentaire dans de multiples maternités avec une centralisation des banques pouvant ainsi être potentiellement éloignées des sites de prélèvements. Cela pourrait également permettre aux banques privées de pouvoir proposer la collecte sur des distances plus éloignées de leur centre de mise en banque. Ainsi et selon un premier aspect, l'invention propose un procédé pour la conservation du sang placentaire entier tel que défini dans la revendication 1. Ce procédé de conservation concerne le sang placentaire entier, c'est-à-dire le sang issu directement d'un cordon ombilical et/un d'un placenta. A part éventuellement l'ajout d'un anticoagulant, ce sang n'a pas été traité et n'a notamment pas subit de traitement de réduction de volume par exemple, par, sédimentation des globules rouges et/ou centrifugation. Le sang n'a pas non plus subit d'étape de congélation. Dans ce procédé, le sang placentaire entier est conservé en l'état liquide, non congelé. Le procédé ne concerne pas non plus les cellules purifiées et/ou isolées du sang de cordon par exemple par gradient de densité.

Il existe essentiellement deux pratiques de prélèvement du sang placentaire. Selon une première pratique, le sang placentaire est prélevé lors de l'accouchement afin que les contractions de la mère aident à l'écoulement du sang dans une poche.

Selon une deuxième pratique, on attend l'expulsion du placenta et, après séparation du cordon de l'enfant, on place le placenta sur une tablette de travail de laquelle le cordon est laissé pendant afin de permettre le recueil du sang placentaire par gravité.

Quelque soit la pratique utilisée, le volume de sang placentaire issu d'un prélèvement est désignée par l'expression "unité de sang placentaire" (USP). Le volume d'une USP est compris entre 50 et 200 ml, notamment entre 80 et 120 ml.

Le procédé de l'invention s'applique au sang placentaire une fois extrait du cordon ombilical et/ou du placenta. Notamment, le procédé s'applique dans les 24 heures suivant le prélèvement du sang placentaire. Au-delà de 24 heures, la perte des cellules d'intérêt devient trop importante.

Les cellules d'intérêt sont notamment les cellules souches hématopoïétiques et les progéniteurs engagés hématopoïétiques qui sont utiles aux reconstitutions hématopoïétiques après une greffe de sang placentaire.

Le procédé de conservation de l'invention est particulièrement destiné à conserver des cellules souches hématopoïétiques et des progéniteurs engagés hématopoïétiques contenus dans le sang placentaire, c'est-à-dire qu'il permet de conserver la viabilité et la fonctionnalité de ces cellules, comme le montre les exemples ci-dessous.

Notamment, le procédé permet d'obtenir, après 3 jours de stockage, un taux de cellules souches hématopoïétiques CD34+ viables d'au moins 80%, particulièrement d'au moins 90%, et encore plus particulièrement proche de 100%, par rapport au nombre de cellules CD34+ viables dans l'unité de sang placentaire juste après le prélèvement.

Aussi, le procédé permet d'obtenir, après 3 jours de stockage, un taux de progéniteurs hématopoïétiques (CFC) viables d'au moins 75%, particulièrement d'au moins 80%, et encore plus particulièrement d'au moins 90% par rapport au nombre de progéniteurs viables dans l'unité de sang placentaire juste après le prélèvement.

Le procédé consiste ainsi à introduire le sang placentaire entier dans une poche de stockage barrière à l'air et à stocker ladite poche contenant le sang placentaire entier.

La poche de stockage est adaptée pour contenir au moins un prélèvement de sang placentaire. Notamment, la poche de stockage a une capacité comprise entre 50 mL et 1 L, en particulier comprise entre 100 mL et 500 mL. Notamment, la poche de stockage possède une capacité de 500 mL.

La poche de stockage barrière à l'air est en particulier une poche comprenant une enveloppe pour stocker le sang placentaire. L'enveloppe est réalisée avec un film barrière à l'air et / ou arrangée dans un emballage réalisé avec un film barrière à l'air. Le film barrière à l'air est un film barrière à l'oxygène ou barrière à l'oxygène et au dioxyde de carbone.

Lorsque la poche comprend un emballage barrière à l'air, l'enveloppe de la poche peut être réalisée dans un film perméable à l'air tel qu'en PVC.

Un "film barrière à l'oxygène" est un film ayant une perméabilité à l'oxygène inférieure à 10 cm³/m².jour.atm. La perméabilité à l'oxygène est déterminée selon la norme ASTM D-3985.

Un "film barrière au dioxyde de carbone" est un film ayant une perméabilité au dioxyde de carbone inférieure à 15 cm³/m².jour.atm. La perméabilité au dioxyde de carbone est déterminée selon la norme ASTM F-2476.

Le film barrière à l'air comprend un polymère barrière à l'air tel que le copolymère d'éthylène-alcool vinylique (EVOH), le copolymère de chlorure de vinylidène, l'alcool polyvinylique, le polyacrylonitrile ou le polyamide.

Le film barrière à l'air est notamment un film comprenant un copolymère d'éthylène-alcool vinylique (EVOH).

En particulier, le film barrière à l'air possède une structure multicouche telle qu'une structure tricouche dont la couche centrale est en matière barrière à l'air. La couche centrale est prise en sandwich entre deux couches d'une autre matière telle qu'une polyoléfine, notamment le polyéthylène, le polypropylène ou un copolymère d'éthylène-oléfine.

Par exemple, le film est un film tricouche en éthylène-acétate de vinyle / éthylène-alcool vinylique / éthylène-acétate de vinyle (EVA/EVOH/EVA).

Ce film barrière à l'air en EVA/EVOH/EVA a la particularité d'offrir une bonne résistance aux techniques de stérilisation par vapeur (chauffage à 121°C pendant 15-20 minutes) et d'être biocompatible avec le contenu de la poche.

Selon une réalisation particulière, l'épaisseur du film formant l'enveloppe de la poche est comprise entre 0,20 et 0,80 mm, notamment 0,50 mm.

Selon une autre réalisation, l'enveloppe de la poche de stockage est réalisée avec un film dont la perméabilité à l'oxygène est de l'ordre de 2,2 cm²/m².jour.atm.

L'enveloppe de la poche de stockage est réalisée avec un film dont la perméabilité au dioxyde de carbone est de l'ordre de 6 cm³/m².jour.atm.

La concentration d'oxygène physiologique dans le sang placentaire varie de 1,1 à 4 % (Kotaska et al, J Clin Lab Anal 24: 300-4). L'utilisation d'une poche barrière à l'air permet de limiter l'apport d'oxygène au sang placentaire, permettant ainsi de rester proche de la concentration d'oxygène physiologique.

Ceci permettrait de préserver la fonctionnalité des cellules d'intérêt du sang placentaire, notamment les cellules souches hématopoïétiques et les progéniteurs engagés.

Avantageusement, le sang placentaire est introduit dans la poche de stockage dans les 24 heures suivant le prélèvement, avant une perte trop importante de la viabilité des cellules d'intérêt.

Plus particulièrement, le sang placentaire est introduit dans la poche de stockage juste après le prélèvement, afin de limiter au maximum l'oxygénation du sang placentaire.

Le stockage de la poche de stockage contenant le sang placentaire est réalisé à une température supérieure à 0°C et inférieure à 40°C, notamment à température ambiante ou à 4°C. Le sang placentaire n'est pas congelé et reste à l'état liquide.

Selon un procédé particulier, la poche est stockée à court terme, c'est-à-dire entre 1 et 14 jours, en particulier entre 1 et 5 jours et plus particulièrement entre 1 et 3 jours.

Dans une forme particulière, le procédé de conservation comprend, avant l'étape de stockage, l'étape d'additionner le sang placentaire à une solution anticoagulante.

La solution anticoagulante est par exemple une solution acide citrate dextrose (ACD) ou citrate phosphate dextrose (CPD).

La quantité de sang placentaire issu d'un prélèvement de sang de cordon varie entre 50 et 200 ml, en moyenne. La quantité de solution anticoagulante à additionner est d'environ 20-30 ml.

Selon une première variante, le sang placentaire est collecté dans une poche de recueil qui contient la solution anticoagulante ou dans laquelle on transfère la solution anticoagulante, de sorte que ce soit le mélange de sang placentaire et de solution anticoagulante qui soit introduit dans la poche de stockage.

En variante, le sang placentaire est introduit dans la poche de stockage qui contient la solution anticoagulante ou dans laquelle on transfère la solution anticoagulante.

Avantageusement, le procédé comprend, avant l'étape de stockage, l'étape d'additionner le sang placentaire avec une solution de conservation. Dans ce cas, la poche de stockage barrière à l'air destinée à être stockée comprend le sang placentaire entier et une solution de conservation.

La solution de conservation est une solution différente de la solution anticoagulante.

L'association d'une poche barrière à l'air et d'une solution de conservation permet d'optimiser les conditions de stockage. Dans ces conditions, le maintien des cellules d'intérêt est proche de 100% des cellules présentes juste après le prélèvement du sang placentaire.
Selon une réalisation particulière, la solution de conservation comprend une solution d'électrolytes, une solution de préservation des cellules ou tissus, ou un milieu de culture.

Par exemple, la solution de conservation comprend une solution d'électrolytes. La solution d'électrolytes comprend par exemple des ions sodium, potassium, calcium, chlorure, zinc, fer, et/ou magnésium.

Une telle solution électrolytique est par exemple la solution Plasmalyte-A®, Normosol-R®, ou la solution de Ringer ou de Ringer lactate.

En variante, la solution de conservation comprend en outre un tampon et/ou un ou plusieurs antioxydants.

Le tampon est choisi par exemple parmi les tampons physiologiques (sulfate, phosphate ou carbonate) ou synthétique (HEPES).

Des exemples d'antioxydants sont des piégeurs de radicaux libres; les chélateurs de fer tels que la déféroxamine; la vitamine E, la vitamine C ou l'érythorbate de sodium; et les dérivés thiolés tels que la N-acétylcystéine, le glutathion ou le glutathion réduit.

Selon une variante, la solution de conservation comprend, en plus de la solution d'électrolytes, un ou plusieurs agents oncotiques. Un agent oncotique est une molécule exerçant une pression oncotique permettant d'éviter l'oedème cellulaire. L'agent oncotique peut être un imperméant tel que le raffinose, le sucrose ou le mannitol et/ou un colloïde tel que l'hydroxyéthylamidon, l'albumine ou le polyéthylène glycol.

En particulier, l'agent oncotique est l'albumine ou un substitut d'albumine. Par exemple, la solution de conservation comprend une solution d'électrolytes, de l'albumine et un tampon.

Dans un autre exemple, la solution de conservation comprend une solution de préservation des cellules et tissus. Une telle solution comprend par exemple une solution d'électrolytes, un agent oncotique et un tampon.

En variante, la solution de préservation des cellules ou tissus comprend en outre des compléments tels que de l'adénosine comme substrat énergétique et/ou un ou plusieurs antioxydants.

Une telle solution de conservation est par exemple la solution Hypothermosol® de Biolife Solutions.

En variante, la solution de conservation comprend, en plus de la solution d'électrolytes, des acides aminés, des vitamines et du glucose.

Par exemple, la solution de conservation comprend un milieu de culture de base. Le milieu de base est un milieu comprenant essentiellement une solution d'électrolytes, des vitamines, des acides aminés, du glucose pour son apport énergétique et un tampon.

A titre d'illustration, le milieu de base est le milieu DMEM (Dulbecco's Modified Eagle's Medium), MEM (Minimal Essential Medium), RPMI 1640, F-10, F-12, αMEM (α Minimal Essential Medium), et IMDM (Iscove's Modified Dulbecco's Medium).

En particulier, le milieu de culture de base comprend en outre des compléments tels que notamment des acides aminés non essentiels, des minéraux, des éléments traces, et/ou
- de l'insuline ou un substitut d'insuline constitué d'un sel de zinc,
- de la transferrine ou un substitut de transferrine comme un chélateur de fer,
- de l'albumine ou un substitut d'albumine comme le polyéthylène glycol (PEG),
- des lipides et/ou acides gras, et/ou
- un ou plusieurs antioxydants.

Dans une forme particulière, la solution de conservation est dépourvue de sérum.

Une solution de conservation particulièrement avantageuse est le milieu de culture MC01 développé par Maco Pharma et commercialisé sous le nom HP02.

Ce milieu comprend :
- un milieu de base commercial comprenant des électrolytes, des vitamines, des acides aminés, du glucose et du pyruvate de sodium pour leur apport énergétique, un tampon HEPES et du rouge de phénol comme indicateur de pH,
- des compléments de base notamment des éléments traces,
- des lipides insolubles,
- de l'insuline humaine recombinante (1-100 mg/L),
- de l'albumine humaine (0,1-0,8 %),
- du gluconate de fer (II) (50-1000 mg/L),
- des antioxydants,
- des compléments spécifiques pour l'expansion de ces cellules tels que des nucléosides (0,1-10 mg/L).

En particulier, le ratio entre la solution de conservation et le sang placentaire dans la poche est compris entre 1:0,5 et 1:2, avantageusement 1:2. La quantité de solution de conservation ne doit pas être trop importante pour ne pas avoir d'effet délétère sur les autres étapes de traitement de l'unité de sang placentaire, notamment l'étape de réduction de volume. Par exemple, pour une USP standard, la quantité de solution de conservation ajoutée est d'environ 50 mL.

Selon un procédé particulier, la solution de conservation est additionnée au sang placentaire avant l'étape de stockage.

Selon une première variante, le sang placentaire est collecté dans une poche de recueil qui contient la solution de conservation ou dans laquelle on transfère la solution de conservation, de sorte que c'est le mélange de sang placentaire et de solution de conservation qui est introduit dans la poche de stockage.

En variante, le sang placentaire est introduit dans la poche de stockage qui contient la solution de conservation ou dans laquelle on transfère la solution de conservation.

Lorsque le procédé comprend l'addition d'une solution anticoagulante et d'une solution de conservation, il est avantageux de
- collecter le sang placentaire dans une poche de recueil contenant la solution anticoagulante,
- additionner la solution de conservation au sang placentaire anticoagulé,
- introduire le sang placentaire anticoagulé additionné de solution de conservation dans la poche de stockage.

Encore plus avantageusement, la solution de conservation est initialement placée dans la poche de stockage barrière à l'air, permettant ainsi le stockage de la solution de conservation avant son utilisation.

Après la conservation de l'unité de sang placentaire selon le procédé décrit ci-dessus, l'unité de sang placentaire est traitée de façon habituelle.

Par exemple, l'unité de sang placentaire subit une étape de réduction de volume ou miniaturisation, notamment à l'aide de l'appareil Sepax (Zingsem J et al., Transfusion, 2003; 43: 806-13).

L'unité de sang placentaire est additionnée d'un agent cryoprotecteur de type DMSO (diméthyl sulfoxyde) pour être congelée et conservée à long terme, c'est-à-dire jusque plusieurs mois, à des températures très négatives, notamment dans l'azote liquide à -196°C.

Lorsqu'une greffe doit être réalisée, l'unité de sang placentaire est décongelée, éventuellement lavée pour éliminer l'agent cryoprotecteur (DMSO), puis injectées au patient.

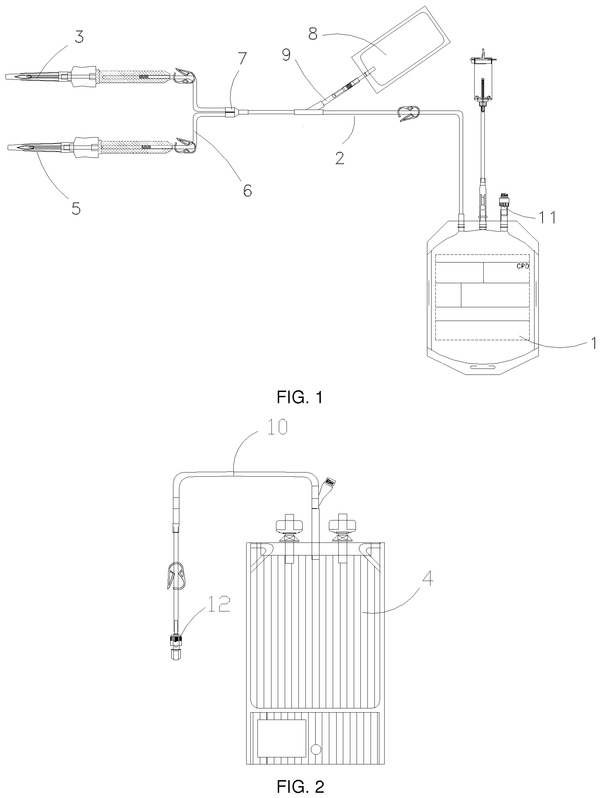

Selon un autre aspect et en relation avec les figures 1 à 3, l'invention concerne un système à poches tel que défini dans la revendication 16. La poche recueil 1 est notamment réalisée en polychlorure de vinyle (PVC). La poche de recueil est connectée par l'intermédiaire d'une première tubulure 2 à une aiguille de prélèvement 3.

Dans une autre réalisation, le système à poches comprend une deuxième aiguille de prélèvement 5 afin de piquer le cordon ombilical à un autre endroit pour récupérer un maximum de sang placentaire.

Dans ce cas, la deuxième aiguille de prélèvement 5 est en communication fluidique par l'intermédiaire d'une deuxième tubulure 6 branchée sur la première tubulure 2 au niveau d'un connecteur 7 en Y.

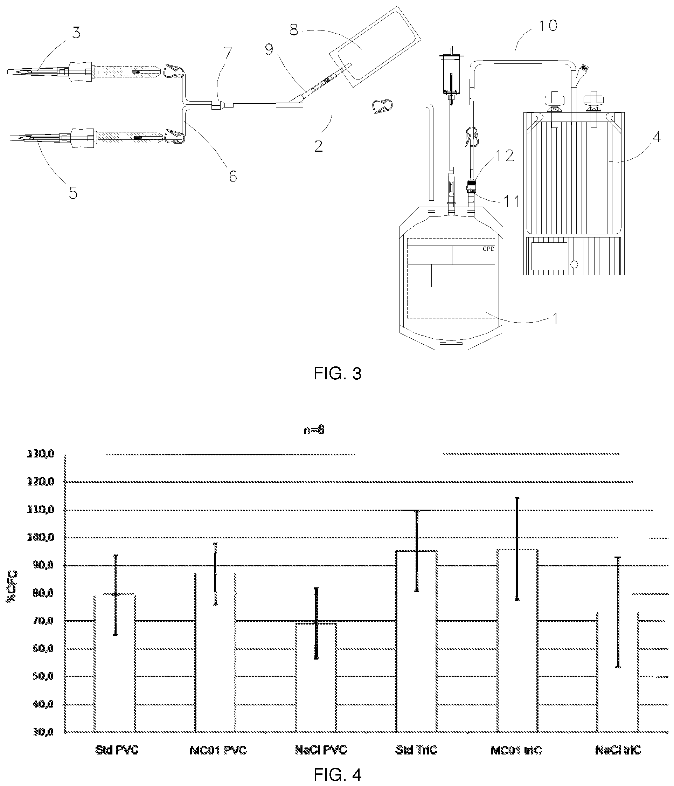

Sur les figures 1 et 3, la poche de recueil 1 contient une solution anticoagulante de type CPD ou ACD afin d'éviter la coagulation du sang placentaire recueilli.

Avantageusement, le système à poches comprend une poche secondaire 8 contenant une solution anticoagulante, différente ou identique à celle de la poche de recueil 1. Cette poche secondaire 8 est en communication fluidique avec la poche de recueil 1 par l'intermédiaire d'une troisième tubulure 9 branchée sur la première tubulure 2.

La solution anticoagulante présente dans cette poche secondaire 8 permet de rincer en partie la première tubulure 2 à la fin de la collecte de sang placentaire afin de récupérer un maximum de sang placentaire. Le volume de solution anticoagulante présente dans cette poche est d'environ 8 ml.

La poche de stockage 4 barrière à l'air est notamment celle décrite ci-dessus en relation avec le procédé de conservation.

Avantageusement, la poche de stockage 4 contient une solution de conservation telle que définie ci-dessus.

Dans une forme particulière représentée sur la figure 3, la poche de stockage 4 est en communication fluidique avec la poche de recueil 1 par l'intermédiaire d'une tubulure 10.

Si la poche de recueil 1 et la poche de stockage 4 sont connectées de fabrication, le système à poches forme un système clos.

En variante, la poche de stockage 4 est destinée à être mise en communication fluidique avec la poche de recueil. Dans ce cas, la poche de recueil 1 et la poche de stockage 4 sont pourvues de moyens de connexion complémentaires 11,12.

Par exemple, la poche de recueil 1 est pourvue au niveau de l'un de ses orifices d'un connecteur 11 de type Luer femelle, et la poche de stockage 4 est pourvue au niveau de l'un de ses orifices d'accès d'une tubulure 10 dont l'extrémité non connectée à la poche de stockage comprend un connecteur 12 de type Luer mâle.

Les tubulures peuvent être munies de clamp afin de permettre ou fermer l'écoulement fluidique entre les différentes poches.

### Exemples utiles à la compréhension de l'invention

Des expériences ont été menées pour étudier la préservation des progéniteurs et des cellules souches hématopoïétiques lors de la conservation d'un sang placentaire dans des poches barrières à l'air. En alternative ou en complément, l'effet de l'ajout d'une solution de conservation a également été testé.

Dans ces exemples, la solution de conservation est le milieu de culture MC01 développé spécifiquement par Maco Pharma. Le sang placentaire est anticoagulé, c'est-à-dire contenant un anticoagulant (CPD). Les poches barrières à l'air sont réalisées avec un film tricouche EVA/EVOH/EVA ayant une perméabilité à l'oxygène de l'ordre de 2,2 cm²/m².jour.atm et une perméabilité au dioxyde de carbone de l'ordre de 6 cm³/m².jour.atm (poche triC).

### Exemple 1 : Tests "miniatures"

### Exemple 1.1 : Effet de l'utilisation de poches barrières à l'air

Un échantillon de sang (8 ml) provenant d'une unité de sang placentaire (USP) ayant moins de 24 h après la naissance a été transféré dans une poche perméable à l'air (PVC) ou barrière à l'air (TriC). Le sang placentaire est ensuite mis au réfrigérateur à +4°C et stocké pendant 3 jour.

Dans certaines conditions, le sang placentaire est additionné de solution de conservation. La proportion de solution de conservation / sang est de 1:2 (4 ml de solution de conservation + 8 ml de sang placentaire). Cette proportion est jugée intéressante pour un développement qui vise une application clinique.

Le sang placentaire a alors été mélangé avec la solution de conservation à son arrivée au laboratoire (<24h) et gardé 3 jours à +4°C.

La série d'expériences a été menée dans les 6 conditions suivantes :
Sdt PVC : 8 ml de sang placentaire dans une poche en PVC
MC01 PVC : 8 ml de sang placentaire + 4 ml de MC01 dans une poche en PVC
NaCl PVC : 8 ml de sang placentaire + 4 ml NaCI dans une poche en PVC
Sdt TriC : 8 ml de sang placentaire dans une poche barrière à l'air
MC01 TriC : 8 ml de sang placentaire + 4 ml de solution MC01 dans une poche barrière à l'air
NaCl TriC : 8 ml de sang placentaire + 4 ml NaCl dans une poche barrière à l'air

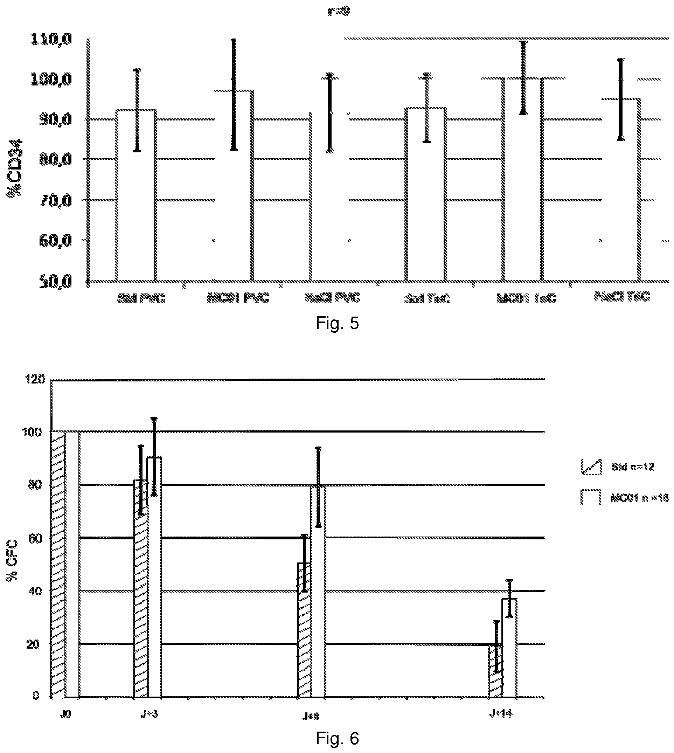

Le nombre de cellules nucléées totales (CNT), de cellules mononuclées (CMN), de cellules CD34+, ainsi que de CFC a été déterminé. Seules les données concernant les CFC et les cellules CD34+ qui sont les plus révélatrices sont présentées (Figures 4 et 5).

Cette série de manipulations a montré un effet positif de l'utilisation de poches barrières à l'air. En effet, le pourcentage de cellules CD34+ et de CFC des USP stockées dans les poches barrières à l'air est toujours supérieur à celui dans les poches en PVC qui sont perméables à l'air. Notamment, l'effet du stockage du sang placentaire dans des poches barrières à l'air sur les CFC, pour lesquelles le pourcentage est supérieur à de 90%, est particulièrement avantageux.

De même, on note que l'ajout d'une solution de conservation a un effet positif, visible sur les cellules CD34+.

Enfin, le maintien des progéniteurs et des cellules CD34+ dans une poche barrière à l'air en combinaison avec une solution de conservation est pratiquement total, c'est-à-dire proche de 100%.

### Exemple 1.2 : Effet de la solution de conservation

Une autre série d'expériences a été effectuée dans des poches en PVC de 30 ml perméables à l'air. Le sang provenant d'une unité de sang placentaire (USP) ayant moins de 24 h après la naissance a été mélangé avec la solution de conservation (10 ml + 10 ml). Pour le contrôle (Std), le sang placentaire ne contient pas de solution de conservation. Le sang placentaire est ensuite mis au réfrigérateur à +4°C et stocké pendant 14 jours. A J0, 3, 8, et 14, on détermine le nombre de cellules totales, des cellules positives au marqueur CD34 (CD34+) et de CFC.

La figure 6 montre la variation du pourcentage de CFC par rapport à J0 au cours de ce stockage (J+3, J+8, J+14). Un effet positif de la solution de conservation sur le maintien de ces progéniteurs fonctionnels à +4°C est visible.

Dans les mêmes conditions de stockage, la capacité des cellules CD34+ conservées deux jours (48 heures) avec ou sans solution de conservation, à s'amplifier ex vivo a été testée. La méthode d'expansion utilisée est décrite dans Ivanovic et al, Cell Transplant 20: 1453-63 2011.

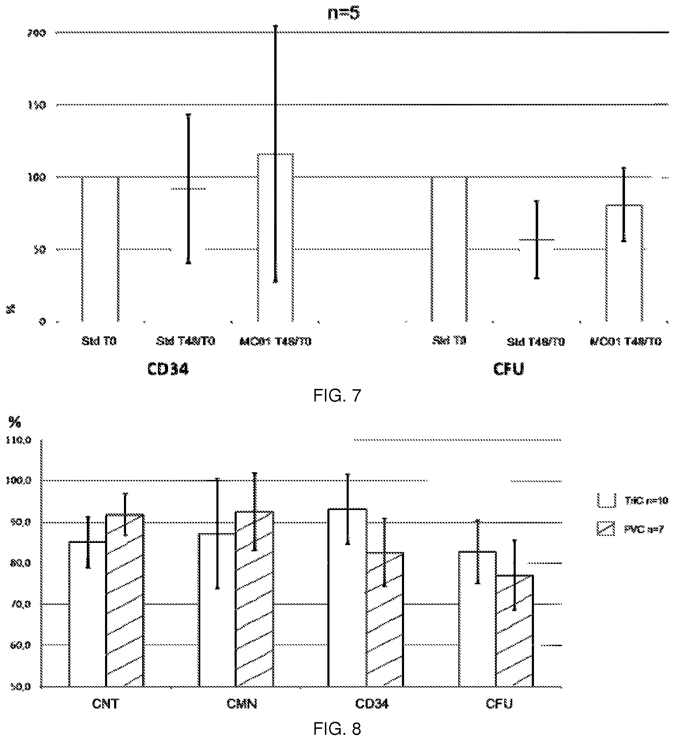

Les résultats (figure 7) montrent que, sans solution de conservation, la capacité d'expansion par rapport à T0 diminue à 50 % à 48h, alors qu'en présence de solution de conservation (MC01), elle se maintient et est significativement élevée par rapport à la condition sans solution de conservation (Std).

### Exemple 3 : Tests "grandeur nature"

Des expériences "grandeur nature", c'est-à-dire avec une USP standard ayant un volume compris entre 80 et 120 ml, ont été menées au cours desquelles le sang placentaire est gardé dans les poches perméables à l'air (PVC) ou barrières à l'air (TriC) pendant 3 jours (Figure 8).

Ici aussi, les cellules d'intérêt (CD34+ et CFC) se maintiennent mieux dans une poche barrière à l'air que dans une poche en PVC, perméable à l'air. Cet effet est plus marqué sur les cellules CD34+ que sur les CFC (contrairement aux résultats des tests "miniatures").

Ainsi, cette série d'expériences démontre que le stockage de sang placentaire entier dans une poche barrière à l'air permet de maintenir les cellules d'intérêt pendant au moins 3 jours à 4°C.

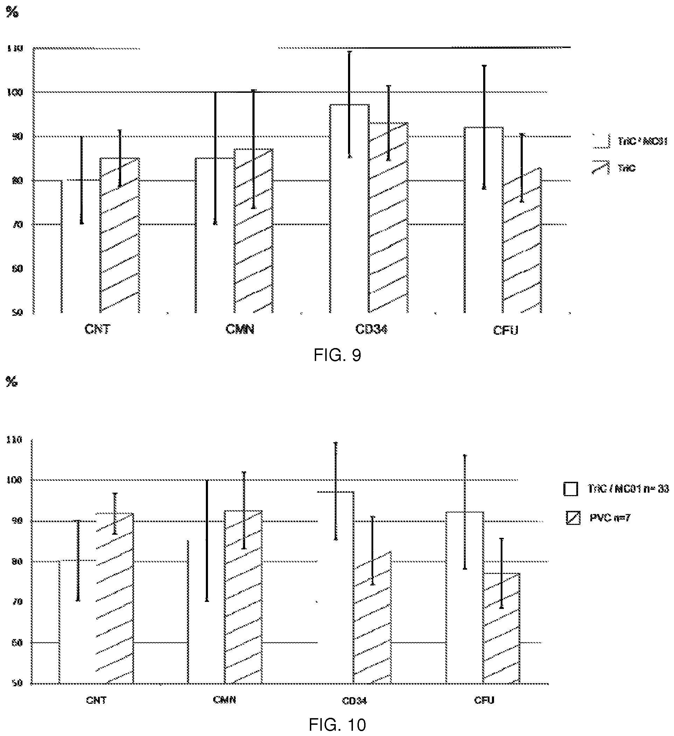

Le maintien de ces cellules pendant 3 jours en poche barrière à l'air avec ou sans solution de conservation (50 ml) a aussi été testé. Cette série de manipulations montre que la présence d'une solution de conservation améliore encore plus le maintien des cellules d'intérêt, notamment les progéniteurs clonogéniques CFC (Figure 9).

La figure 10 compare le maintien du sang placentaire à +4°C à 3 jours dans les conditions de routine (poches PVC perméables à l'air sans solution de conservation) et dans les conditions les plus avantageuses de l'invention (poches barrières à l'air et solution de conservation).

Les résultats de cette série d'expériences sont clairement visibles : la combinaison d'une poche barrière à l'air avec la solution de conservation permet un maintien des CFC et des cellules CD34+ pour 1/3 supérieur par rapport aux poches standards sans solution de conservation. Ceci suggère sans équivoque l'intérêt de l'association de solution de conservation avec les poches barrières à l'air pour préserver les progéniteurs fonctionnels.

Cette série d'expériences permet donc de constater que le principe de protection des cellules par empêchement/limitation de leur hyperoxygénation peut opérer à + 4°C sur le sang placentaire sans séparation préalable des cellules.

De plus, l'association de poches barrières à l'air et d'une solution de conservation peut maintenir pratiquement la totalité des progéniteurs hématopoïétiques pendant 3 jours à +4°C alors qu'avec les poches perméables à l'air et sans solution de conservation, ce maintien se situe aux alentours de 85% pour les CD34+ et 75% pour les progéniteurs clonogéniques.

### Exemple 4 : Etude pré-clinique

Afin d'évaluer le procédé de conservation dans une situation s'approchant le plus possible de la réalité d'une utilisation routinière, une étude a été entreprise pour étudier le nombre absolu de progéniteurs (CFC), ainsi que l'activité des cellules souches (SRC) hématopoïétiques avant et après chaque étape de traitement du sang placentaire. Ce traitement comprend :
- un stockage à 3 jours (J3),
- une réduction en volume (appelée aussi « miniaturisation ») en utilisant la technologie Sépax, (AS).
- la congélation contrôlée (technologie Bioarchive)
- la décongélation (AD), et
- l'état fonctionnel des cellules 6 heures après décongélation (AD+6h).

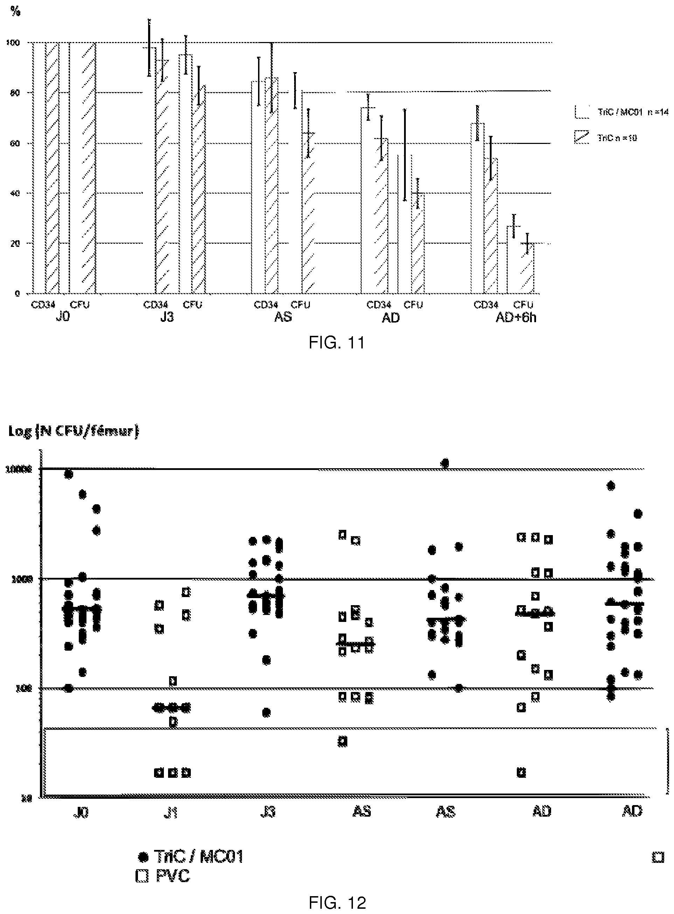

La comparaison du maintien des cellules CD34+ et des CFC après 3 jours de stockage, ainsi qu'après chaque étape, dans une poche barrière à l'air, avec ou sans ajout de 50 ml de solution de conservation, mentionnée ci-dessus, est présentée sur la Figure 11.

Il est évident que l'association de poches barrières à l'air et de la solution de conservation permet de maintenir la totalité des cellules CD34+ et des progéniteurs hématopoïétiques (ces derniers se situent à 80% sans milieu après 3 jours de stockage). Cet avantage qui concerne le maintien des progéniteurs (CFC) est bien maintenu après la miniaturisation par Sépax ainsi qu'après décongélation des échantillons.

Ces résultats montrent donc qu'au niveau de l'effet biologique, le procédé de l'invention est compatible avec un ajout de milieu à la maternité. Ceci signifie qu'avec un tel procédé, un meilleur maintien des progéniteurs hématopoïétiques peut être assuré jusqu'à 72 heures après le prélèvement à +4°C, ce qui devrait permettre un transport des USP jusqu'à la banque quelle que soit la distance entre la maternité et la banque. Il confirme également que l'avantage gagné est maintenu tout au long du processus de cryopréservation du sang placentaire jusqu'au produit décongelé.

Exemple 5 : Etude de préservation des cellules souches en utilisant l'approche in vivo (sur les souris NOG/Scid).

A la différence des progéniteurs engagés, qui participent à la première vague des reconstitutions hématopoïétiques après la greffe sans être en mesure d'assurer un effet à long terme, les cellules souches prennent le relais un peu plus tard mais leur effet est à long terme assurant donc la longévité du greffon.

L'approche expérimentale pour mettre en évidence ces cellules souches comprend un test in vivo : greffe des souris immuno-déficientes (ne rejetant pas les cellules humaines). Des souris NOG/Scid sont utilisées. Ces souris ont un meilleur seuil de greffe que les souris NOD/Scid utilisées auparavant. Brièvement, les souris sont d'abord conditionnées avec du busulfan. Les cellules humaines sont injectées 2 jours après le conditionnement, et la prise de greffe est étudiée 8 semaines après. Chaque souris a reçu une dose de cellules CD34+ qui provient de la même fraction d'unité de sang placentaire que celle contenant 1000 cellules CD34+ à T0. Ainsi, les résultats sont directement comparables sans aucun calcul de conversion.

Après avoir sacrifié les souris 8 semaines après la greffe, la présence dans la moelle osseuse de cellules d'origine humaine positives pour CD45, CD33 et CD19 est étudiée. On a également étudié le contenu en progéniteurs hématopoïétiques (CFC) d'origine humaine par implantation de ces cellules (suspension cellulaire récupérée de la moelle osseuse des souris) dans les cultures en méthylcellulose avec des facteurs de croissance spécifiques aux cellules humaines. Pour des raisons de simplicité, et à cause de la nature fonctionnelle de ce dernier test, seuls les résultats basés sur cette détection de progéniteurs humains dans la moelle osseuse des souris sont montrés.

Ces résultats (Figure 12) montrent un maintien absolu des SRC conservées 3 jours dans une poche barrière à l'air avec la solution de conservation tout au long du traitement des USP jusqu'à leur décongélation. Ce maintien semble être supérieur à celui d'une USP miniaturisée et congelée après 24 h dans une poche perméable à l'air (PVC) sans solution de conservation (condition qui convient à la routine).

Ces résultats montrent donc que l'approche consistant à utiliser des poches barrières à l'air et une solution de conservation permet de prolonger le maintien des progéniteurs et des cellules souches hématopoïétiques d'un sang placentaire à 72h (3 jours) sans aucune perte d'activité de ces populations critiques par rapport à un stockage simple de 24 heures dans les poches PVC-c'est-à-dire la routine actuelle.

## Revendications

1. Procédé pour la conservation du sang placentaire entier comprenant les étapes de :
- introduire du sang placentaire entier dans une poche de stockage barrière à l'air, ladite poche de stockage étant réalisée avec un film dont la perméabilité à l'oxygène est inférieure à 10 cm³/m².jour.atm,
- stocker ladite poche contenant le sang placentaire entier à une température supérieure à 0°C et inférieure à 40°C, de sorte à conserver le sang placentaire entier.

2. Procédé selon la revendication 1, **caractérisé en ce que** le sang placentaire est introduit dans la poche de stockage dans les 24 heures suivant le prélèvement.

3. Procédé selon l'une quelconque des revendications 1 à 2, **caractérisé en ce qu'**il comprend, avant l'étape de stockage, l'étape d'additionner le sang placentaire à une solution anticoagulante.

4. Procédé selon la revendication 3, **caractérisé en ce que** la solution anticoagulante est la solution acide citrate dextrose (ACD) ou citrate phosphate dextrose (CPD).

5. Procédé selon l'une quelconque des revendications 1 à 4, **caractérisé en ce qu'**il comprend, avant l'étape de stockage, l'étape d'additionner le sang placentaire avec une solution de conservation.

6. Procédé selon la revendication 5, **caractérisé en ce que** la solution de conservation comprend une solution d'électrolytes.

7. Procédé selon l'une des revendications 5 ou 6, **caractérisé en ce que** la solution de conservation comprend un ou plusieurs agents oncotiques.

8. Procédé selon la revendication 7, **caractérisé en ce que** l'agent oncotique est l'albumine ou un substitut d'albumine.

9. Procédé selon l'une quelconque des revendications 5 à 8, **caractérisé en ce que** la solution de conservation comprend des acides aminés, des vitamines et du glucose.

10. Procédé selon l'une quelconque des revendications 5 à 9, **caractérisé en ce que** la solution de conservation comprend un ou plusieurs antioxydants.

11. Procédé selon l'une quelconque des revendications 5 à 10, **caractérisé en ce que** le ratio entre la solution de conservation et le sang placentaire dans la poche est compris entre 1:0,5 et 1:2.

12. Procédé selon l'une quelconque des revendications 1 à 11, **caractérisé en ce que** la poche est stockée entre 1 et 14 jours.

13. Procédé selon l'une quelconque des revendications 1 à 12, **caractérisé en ce que** ladite poche de stockage comprend une enveloppe pour stocker le sang placentaire, ladite enveloppe étant réalisée avec un film barrière à l'air et / ou arrangée dans un emballage réalisé avec un film barrière à l'air.

14. Procédé selon la revendication 13, **caractérisé en ce que** le film barrière à l'air possède une structure tricouche dont la couche centrale en matière barrière à l'air est prise en sandwich entre deux couches d'une autre matière.

15. Procédé selon l'une des revendications 13 ou 14, **caractérisé en ce que** le film barrière à l'air est un film tricouche en éthylène-acétate de vinyle / éthylène-alcool vinylique / éthylène-acétate de vinyle (EVA/EVOH/EVA).

16. Système à poches pour la mise en oeuvre d'un procédé selon la revendication 1, ledit système comprenant :
- au moins une aiguille de prélèvement (3),
- une poche de recueil du sang placentaire (1) en communication fluidique, par l'intermédiaire d'une première tubulure (2) associée à un orifice d'entrée de la poche de recueil, avec ladite au moins une aiguille de prélèvement,
- une poche de stockage barrière à l'air (4), ladite poche de stockage étant réalisée avec un film dont la perméabilité à l'oxygène est inférieure à 10 cm³/m².jour.atm, ladite poche étant en communication fluidique ou destinée à être mise en communication fluidique avec la poche de recueil pour pouvoir stocker le sang placentaire.

17. Système à poches selon la revendication 16, **caractérisé en ce que** la poche de recueil contient un anticoagulant.

18. Système à poches selon la revendication 16 ou 17, **caractérisé en ce que** la poche de stockage contient une solution de conservation.

19. Système à poches selon l'une quelconque des revendications 16 à 18, **caractérisé en ce que** la poche de stockage comprend une enveloppe pour stocker le sang placentaire, ladite enveloppe étant réalisée avec un film barrière à l'air et / ou arrangée dans un emballage réalisé avec un film barrière à l'air.

20. Système à poches selon la revendication 19, **caractérisé en ce que** le film barrière à l'air possède une structure tricouche dont la couche centrale en matière barrière à l'air est prise en sandwich entre deux couches d'une autre matière.

21. Système à poches selon l'une des revendications 19 ou 20, **caractérisé en ce que** le film barrière à l'air est un film tricouche en éthylène-acétate de vinyle / éthylène-alcool vinylique / éthylène-acétate de vinyle (EVA/EVOH/EVA).

## Patentansprüche

1. Verfahren zur Konservierung des Plazentavollblutes, die Schritte umfassend:
- Einleiten des Plazentavollblutes in einen Aufbewahrungsbeutel mit Luftsperre, wobei der besagte Aufbewahrungsbeutel aus einer Folie gefertigt ist, deren Sauerstoffdurchlässigkeit geringer ist, als 10 cm³/m².Tag.atm.,
- Aufbewahren des besagten Beutels, der das ganze Plazentablut enthält, bei einer Temperatur über 0°C und unter 40°C, um das Plazentavollblut zu konservieren.

2. Verfahren nach Anspruch 1, **dadurch gekennzeichnet, dass** das Plazentablut innerhalb von 24 Stunden nach der Entnahme in den Aufbewahrungsbeutel eingeleitet wird.

3. Verfahren nach einem der Ansprüche 1 bis 2, **dadurch gekennzeichnet, dass** es vor dem Schritt des Aufbewahrens den Schritt des Beigebens des Plazentablutes zu einer Antikoagulanslösung umfasst.

4. Verfahren nach Anspruch 3, **dadurch gekennzeichnet, dass** die Antikoagulanslösung die Acid-Citrat-Dextrose (ACD) oder Citrat-Phosphat-Dextrose (CPD) Lösung ist.

5. Verfahren nach einem der Ansprüche 1 bis 4, **dadurch gekennzeichnet, dass** es vor dem Schritt des Aufbewahrens den Schritt des Beigebens des Plazentablutes zu einer Konservierungslösung umfasst.

6. Verfahren nach Anspruch 5, **dadurch gekennzeichnet, dass** die Konservierungslösung eine Elektrolytlösung umfasst.

7. Verfahren nach einem der Ansprüche 5 oder 6, **dadurch gekennzeichnet, dass** die Konservierungslösung ein oder mehrere onkotische Mittel umfasst.

8. Verfahren nach Anspruch 7, **dadurch gekennzeichnet, dass** das onkotische Mittel Albumin oder ein Albumin-Ersatz ist.

9. Verfahren nach einem der Ansprüche 5 bis 8, **dadurch gekennzeichnet, dass** die Konservierungslösung Aminosäuren, Vitamine und Glukose umfasst.

10. Verfahren nach einem der Ansprüche 5 bis 9, **dadurch gekennzeichnet, dass** die Konservierungslösung eines oder mehrere Antioxidantien umfasst.

11. Verfahren nach einem der Ansprüche 5 bis 10, **dadurch gekennzeichnet, dass** das Verhältnis zwischen der Konservierungslösung und dem Plazentablut im Beutel zwischen 1:0,5 und 1:2 enthalten ist.

12. Verfahren nach einem der Ansprüche 1 bis 11, **dadurch gekennzeichnet, dass** der Beutel zwischen 1 und 14 Tage aufbewahrt wird.

13. Verfahren nach einem der Ansprüche 1 bis 12, **dadurch gekennzeichnet, dass** der besagte Aufbewahrungsbeutel eine Hülle zum Aufbewahren des Plazentablutes umfasst, wobei die besagte Hülle aus einer Folie mit Luftsperre gefertigt ist und/ oder in einer Verpackung angeordnet ist, die aus einer Folie mit Luftsperre gefertigt ist.

14. Verfahren nach Anspruch 13, **dadurch gekennzeichnet, dass** die Folie mit Luftsperre eine dreischichtige Struktur besitzt, deren mittlere Schicht aus einem Luftsperre-Material ist und zwischen zwei Schichten aus einem anderen Material eingeschlossen ist.

15. Verfahren nach einem der Ansprüche 13 oder 14, **dadurch gekennzeichnet, dass** die Folie mit Luftsperre eine dreischichtige Folie aus Ethylen- Vinylacetat / Ethylen-Vinylalkohol / Ethylen- Vinylacetat (EVA/EVOH/EVA) ist.

16. System mit Beuteln für die Anwendung eines Verfahrens nach Anspruch 1, wobei das besagte System umfasst:
- zumindest eine Entnahmenadel (3),
- einen Beutel zum Aufnehmen von Plazentablut (1) in Fluidverbindung über einen ersten Stutzen (2), der einer Eingangsöffnung des Beutels zum Aufnehmen mit der besagten zumindest einen Entnahmenadel zugeordnet ist,
- einen Aufbewahrungsbeutel mit Luftsperre (4), wobei der besagte Aufbewahrungsbeutel aus einer Folie gefertigt ist, deren Sauerstoffdurchlässigkeit geringer ist, als 10 cm³/m².Tag.atm., wobei der besagte Beutel in Fluidverbindung ist oder dazu bestimmt ist, in Fluidverbindung mit dem Beutel zum Aufnehmen gebracht zu werden, um das Plazentablut aufbewahren zu können.

17. System mit Beuteln nach Anspruch 16, **dadurch gekennzeichnet, dass** der Beutel zum Aufnehmen ein Antikoagulans enthält.

18. System mit Beuteln nach Anspruch 16 oder 17, **dadurch gekennzeichnet, dass** der Aufbewahrungsbeutel eine Konservierungslösung enthält.

19. System mit Beuteln nach einem der Ansprüche 16 bis 18, **dadurch gekennzeichnet, dass** der Aufbewahrungsbeutel eine Hülle zum Aufbewahren des Plazentablutes umfasst, wobei die besagte Hülle aus einer Folie mit Luftsperre gefertigt ist und/ oder in einer Verpackung angeordnet ist, die aus einer Folie mit Luftsperre gefertigt ist.

20. System mit Beuteln nach Anspruch 19, **dadurch gekennzeichnet, dass** die Folie mit Luftsperre eine dreischichtige Struktur besitzt, deren mittlere Schicht aus einem Luftsperre-Material ist und zwischen zwei Schichten aus einem anderen Material eingeschlossen ist.

21. System mit Beuteln nach einem der Ansprüche 19 oder 20, **dadurch gekennzeichnet, dass** die Folie mit Luftsperre eine dreischichtige Folie aus Ethylen- Vinylacetat / Ethylen-Vinylalkohol / Ethylen- Vinylacetat (EVA/EVOH/EVA) ist.

## Claims

1. Method for preserving whole placental blood including steps to:
- introduce whole placental blood into an air barrier storage bag, said storage bag being made with a film with permeability to oxygen of less than 10cm³/m².day.atm,
- store said bag containing whole placental blood at a temperature of more than 0°C and less than 40°C, so as to preserve the whole placental blood.

2. Method according to claim 1, **characterised in that** placental blood is introduced into the storage bag within 24 hours after removal.

3. Method according to any one of claims 1 to 2, **characterised in that** it includes, before the storage step, a step for adding placental blood to an anticoagulant solution.

4. Method according to claim 3, **characterised in that** the anticoagulant solution is the acid citrate dextrose (ACD) solution or the citrate phosphate dextrose (CPD) solution.

5. Method according to any one of claims 1 to 4, **characterised in that**, before the storage step, the method includes a step to add placental blood with a preservation solution.

6. Method according to claim 5, **characterised in that** the preservation solution includes a solution of electrolytes.

7. Method according to either claim 5 or 6, **characterised in that** the preservation solution comprises one or several oncotic agents.

8. Method according to claim 7, **characterised in that** oncotic agent is albumin or an albumin substitute.

9. Method according to any one of claims 5 to 8, **characterised in that** the preservation solution comprises amino acids, vitamins and glucose.

10. Method according to any one of claims 5 to 9, **characterised in that** the preservation solution comprises one or several antioxidants.

11. Method according to any one of claims 5 to 10, **characterised in that** the ratio between the preservation solution and placental blood in the bag is comprised between 1:0.5 and 1:2.

12. Method according to any one of claims 1 to 11, **characterised in that** the bag is stored for between 1 and 14 days.

13. Method according to any one of claims 1 to 12, **characterised in that** said storage bag comprises an envelope for storing placental blood, said envelope being made with an air barrier film and/or being arranged in a packaging made with an air barrier film.

14. Method according to claim 13, **characterised in that** the air barrier film has a three-layer structure in which the central layer made from an air barrier material is sandwiched between two layers made from another material.

15. Method according to either claim 13 or 14, **characterised in that** the air barrier film is a three-layer film made of ethylene vinyl acetate / ethylene vinyl alcohol / ethylene vinyl acetate (EVA/EVOH/EVA).

16. System of bags for implementation of a method according to claim 1, said system comprising:
- at least one draw-off needle (3),
- a placental blood collection bag (1) in fluid communication, through a first tube (2) associated with an inlet orifice of the collection bag, with said at least one draw-off needle,
- an air barrier storage bag (4), said storage bag being made with a film with permeability to oxygen of less than 10cm³/m².day.atm, said bag being in fluid communication or intended to be brought into fluid communication with the collection bag, so that the placental blood can be stored.

17. System of bags according to claim 16, **characterised in that** the collection bag contains an anticoagulant.

18. System of bags according to either claim 16 or 17, **characterised in that** the collection bag contains a preservation solution.

19. System of bags according to any one of claims 16 to 18, **characterised in that** the collection bag comprises an envelope for storing placental blood, said envelope being made with an air barrier film and/or being arranged in a packaging made with an air barrier film.

20. System of bags according to claim 19, **characterised in that** the air barrier film has a three-layer structure in which the central layer made from an air barrier material is sandwiched between two layers made from another material.

21. System of bags according to either claim 19 or 20, **characterised in that** the air barrier film is a three-layer film made of ethylene vinyl acetate / ethylene vinyl alcohol / ethylene vinyl acetate (EVA/EVOH/EVA).
